# EUROPEAN PATENT APPLICATION

(11) **EP 4 850 698 A1**
(43) Date of publication of application: **09.12.2026**
(21) Application number: 25747928.7
(22) Date of filing: 26.01.2025
(51) Int. Cl.: A61K 47/64, A61K 47/68, A61P 35/00, C07H 1/00

(54) **TROP2-TARGETED ANTIBODY-DRUG CONJUGATE, AND PREPARATION METHOD THEREFOR AND USE THEREOF**

(30) Priority: 02.02.2024 CN 202410156204
(71) Applicant: Shanghai Tangling Biomedical Co., Ltd., Shanghai 201210 (CN); Shanghai Institute of Materia Medica, Chinese Academy of Sciences, Shanghai 201203 (CN)
(72) Inventor: HUANG, Wei, Shanghai 201203 (CN); TANG, Feng, Shanghai 201203 (CN); SHI, Wei, Shanghai 201203 (CN); YANG, Yang, Shanghai 201210 (CN); LUO, Caili, Shanghai 201210 (CN); TANG, Caihong, Shanghai 201210 (CN); ZHANG, Jianxin, Shanghai 201210 (CN)
(74) Representative: Witte, Weller & Partner Patentanwälte mbB
(86) International application number: PCT/CN2025/075118
(87) International publication number: WO 2025/162361

(57) **Abstract**

Disclosed are a TROP2-targeted antibody-drug conjugate (ADC), and a preparation method therefor and a use thereof. The TROP2-targeted ADC has a structure as shown in formula I. In the present invention, glycosyl site-directed technology is used to specifically link the cell toxins MMAE and Eribulin, and the like to an antibody glycosylation site, and an obtained ADC compound has good in-vitro uniformity and toxicity for TROP2-positive cells, and can be used for preparing anti-tumor drugs for TROP2-positive tumors.

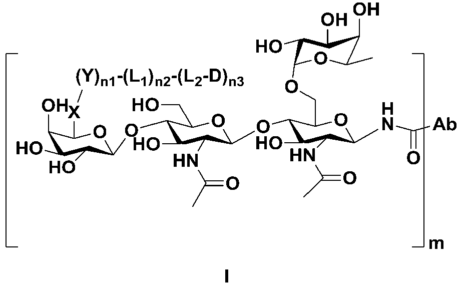

## Description

### TECHNICAL FIELD

The present invention belongs to the fields of medicinal chemistry and biotechnology drugs, specifically relating to a sugar site-specific conjugation technology, and more specifically to a class of TROP2-targeting antibody-drug conjugates, their preparation methods and uses.

### BACKGROUND ART

Trop2 (Tumor-associated calcium signal transducer 2, also known as TACSTD2, EGP-1, GA733-1) is a single-pass transmembrane glycoprotein encoded by the TACSTD2 gene located on cell surface. It is highly expressed in lung adenocarcinoma and triple-negative breast cancer, and is associated with tumor growth, metastasis, and patient poor prognosis, making it a target with clinical development value.

Antibody-drug conjugates (ADCs), which are complexes formed by chemically conjugating antibodies to highly cytotoxic small molecules (payloads), can target and deliver cytotoxic small molecules to cancer cells. So far, more than a dozen ADC drugs have been approved for marketing. Among others, Sacituzumab govitecan (SG, IMMU-132), which is a novel Trop2-targeting ADC drug developed by Immunomedics, Inc., consists of a humanized antibody hRS7, a maleimide-polyethylene glycol-acid-sensitive cleavable carbonate linker, and an irinotecan metabolite (SN-38), and is approved for the treatment of metastatic triple-negative breast cancer and urothelial carcinoma. In addition, several Trop2-targeting ADCs are in clinical trials, for example, Datopotamab deruxtecan (Dato-Dxd, DS-1062a) is a Trop2-targeting ADC developed by Daiichi Sankyo Co., Ltd., wherein the Topo I inhibitor Dxd is randomly conjugated to the Datopotamab antibody. RN927C, which realizes a site-specific modification of AcLys-VC-PF-06380101 at the C-terminus of the heavy chain of a humanized anti-Trop2 IgG1 antibody (PF-06478924, RN926) via enzymatic reaction to produce a highly homogeneous conjugate, was terminated in a Phase I trial due to overdose toxicity and limited objective tumor response. SKB264, an investigational Trop2-targeting ADC drug from Kelun Biotech, is currently in Phase III clinical trials. It has a monoclonal antibody that is the same as IMMU-132 and uses a novel drug linker intermediate TL033 to conjugate the payload KL610023 (T030, a beloteccan derivative, which is a topoisomerase I inhibitor that, upon internalization, arrests the cell cycle at the G2/S stage, leading to cell death) at the cysteine site and its drug-antibody ratio is 7-8.

Most of the aforementioned marketed or investigational Trop2-targeting ADCs employ a random conjugation strategy, i.e., modifying the drug-linker by partially reducing interchain disulfide bonds in antibodies to expose reactive thiol groups. Although this method is simple to operate, it still has the following shortcomings: 1) The reduction of interchain disulfide bonds in antibodies may affect the stability of ADC products; 2) The homogeneity of these ADC products is poor; 3) The structure formed by maleimide and thiol may undergo anti-Markovnikov addition, leading to poor plasma stability and possible off-target toxicity; 4) The strong hydrophobicity of the toxin small molecule may lead to insufficient ADC aggregation stability. To improve the homogeneity and stability of ADCs, site-specific conjugation technology has become the preferred method for next-generation ADCs.

Furthermore, Trop2 targets are widely distributed throughout the body. Currently, most marketed or clinically investigated Trop2-targeting ADC drugs use DNA topoisomerase inhibitors as cytotoxic agents. Microtubule inhibitors such as MMAE have strong toxicity, which can lead to on-target toxicity. Most ADC compounds using MMAE as a toxin have been discontinued in preclinical and clinical stages due to safety concerns.

### SUMMARY OF THE INVENTION

Based on the problems existing in the prior art, the present invention aims to provide an TROP2-targeting antibody-drug conjugate with high stability, high homogeneity, high tumor inhibiting activity, and high safety, and its preparation method and uses.

To achieve the above aim the technical solutions provided in this application are as follows. In the first aspect, the present invention provides an TROP2-targeting antibody-drug conjugate having a structure shown in the following Formula I: 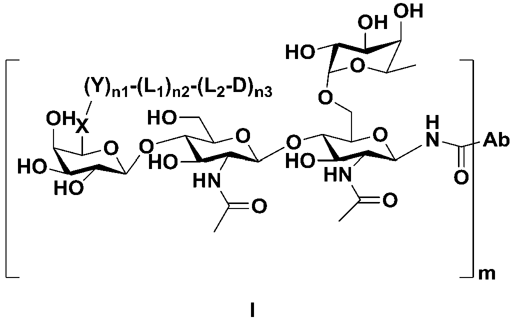
wherein m is 1 or 2, preferably 2;
Ab represents a TROP2-targeting antibody, which is an IgG having a conserved N-glycosylation site at N297 in the Fc region;
Xs are each independently -(CH=N-O)ₚ₀-(CH₂)ₚ₁; wherein p0 is 0 or 1, and p1 is an integer between 1 and 20, preferably an integer between 1 and 6, for example, 2;
Y represents -W-L₃-, wherein W represents a structure formed by a reaction of orthorhombic groups to form a stable covalent bond, and the orthorhombic groups are selected from azide (N₃) group, dibenzocyclooctyl (DBCO), bicyclo[6.1.0]nonyne (BCN) group, and dibenzooctylene (DIBO) group, etc.; L₃ represents a linking group selected from -(CH₂)ₚ₂-CO-, -(CH₂CH₂O)ₚ₃-CO-, and -(CH₂)ₚ₄-CONH-(CH₂CH₂O)ₚ₅-(CH₂)ₚ₆-CO-; wherein p2, p3, p4, p5, and p6 are each independently selected from an integer between 1 and 20, preferably an integer between 2 and 6, such as 2 and 4;
n1 is 0 or 1;
L₁ represents a linking group having 3 or more linking sites, such as a linking group derived from glutamic acid (Glu), β-glutamic acid, lysine (Lys), Lys-Glu dipeptide, or lysine-β-glutamic acid dipeptide, or a linking group derived from diethanolamine;
L₂ represents a short peptide linear linking group with 2 or more linking sites, with or without p-aminobenzylcarbamate, for example, it is a structural unit that is cleavable or non-cleavable in tumor microenvironment, such as Val-Cit-PAB, Val-Ala-PAB, Gly-Gly-Phe-Gly, or the like; further, optionally, L₁ and L₂ have a PEG chain attached to the end of their respective groups contained therein, for example, when L₁ is a linking group derived from Lys, a PEG chain is further attached to the amino group of lysine via an amide bond, and the PEG chain is, for example, -(CH₂CH₂O)ₚ₇-CH₃, wherein p7 is an integer between 2 and 20, such as 5, 6, 7, 8, 9, 10, 11, 12, 13, or 14;
D represents a group derived from a small molecule toxin drug;
n2 is 0 and n3 is 1; or n2 is 1 and n3 is an integer between 2 and 4, for example, n2 is 1 and n3 is 2, 3, or 4, preferably n3 is 2;
wherein n2 is 1, n3 of L₂-D groups are covalently linked to L₁, and n3 ≤ the number of linking sites of L₁ - 1.

In some embodiments, Ab is an antibody selected from IgG1, IgG2, or IgG4, preferably IgG1; Specifically, the antibody is a monoclonal antibody, a polyclonal antibody, a bifunctional antibody, a trifunctional antibody, a nanobody fused with an Fc domain, or a therapeutic or functional antibody from different species (mouse, rat, sheep, rabbit, human, etc.).

Specifically, the antibody includes Sacituzumab and Datopotamab, preferably Sacituzumab.

In some embodiments, W is selected from

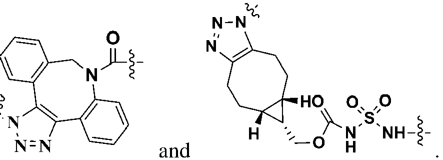

In some embodiments, L₁ represents a linking group derived from glutamic acid (Glu), lysine (Lys), or diethanolamine; preferably, L₁ is selected from 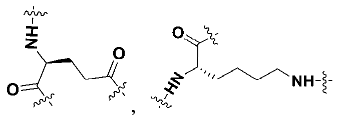 and

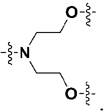

In some embodiments, L₂ is

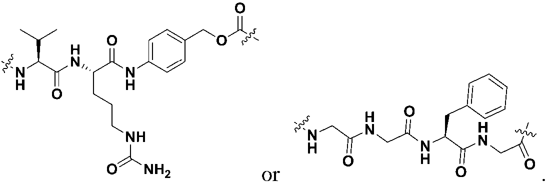

In some embodiments, the toxin small molecule includes MMAE, MMAF, Eribulin or Dxd, Exatecan, DM1, DM4, etc.; preferably, the toxin small molecule is a microtubule inhibitor MMAE or Eribulin.

Specifically, D is selected from the following structures:

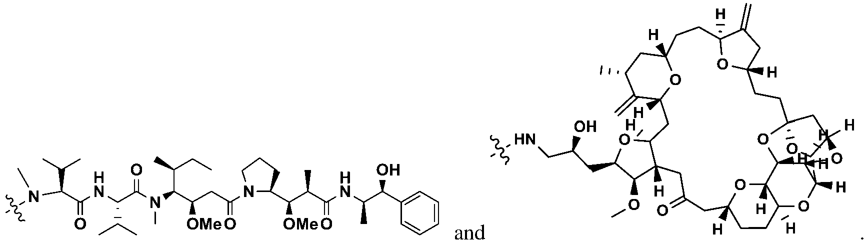

In some embodiments, the TROP2-targeting antibody-drug conjugate has a structure shown in the following Formula **I-1 or** Formula **I-2:** 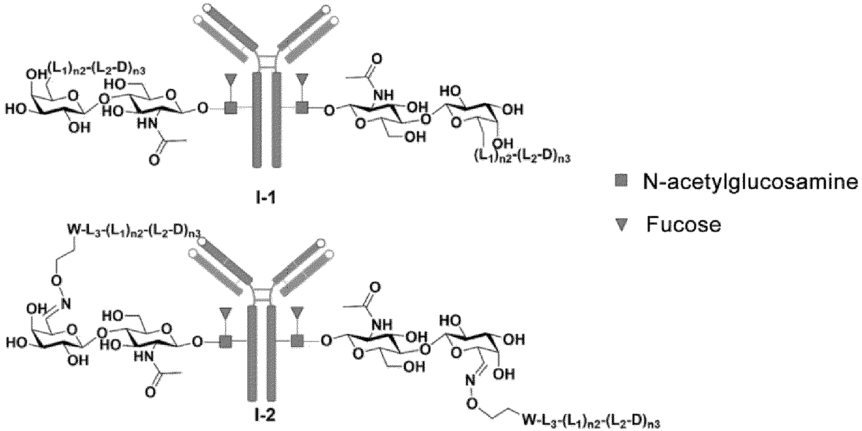 wherein W, L₁, L₂, L₃, D, n2, n3, and the antibody are each defined as described above.

In some embodiments, the TROP2-targeting antibody-drug conjugate has a DAR value of more than 0 and 4 or less, preferably 0.5 or more and 4 or less, more preferably, the DAR value is 1 to 4, and even more preferably, the DAR value is 2 or 4.

In some embodiments, the TROP2-targeting antibody-drug conjugate has the following structure:

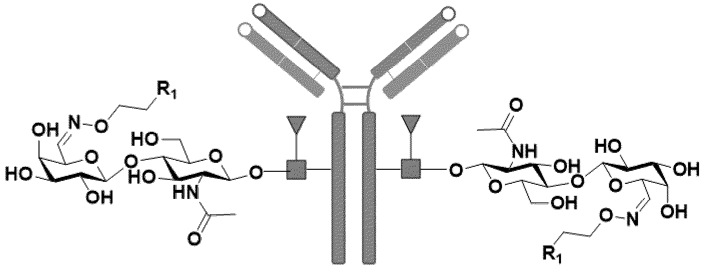

| ADC No. | R₁ |
|---|---|
| ADC-1 | 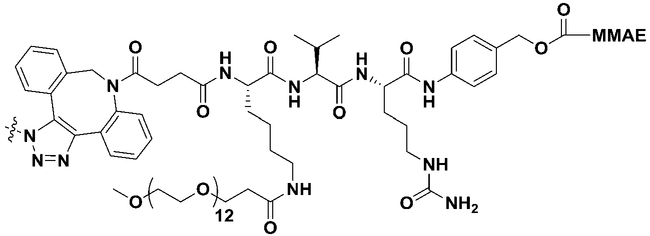 |
| ADC-2 | 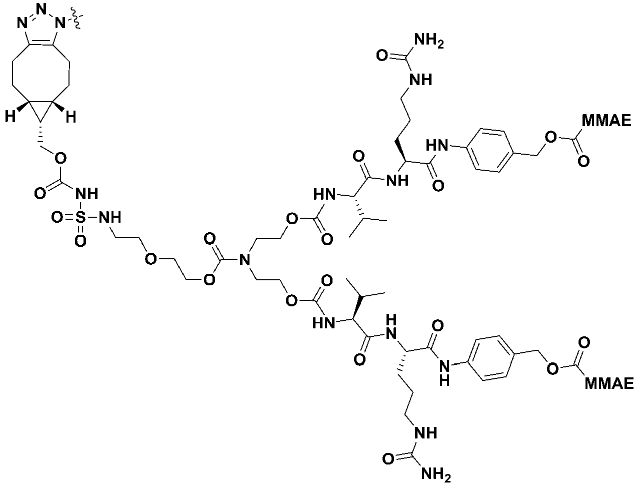 |
| ADC-3 | 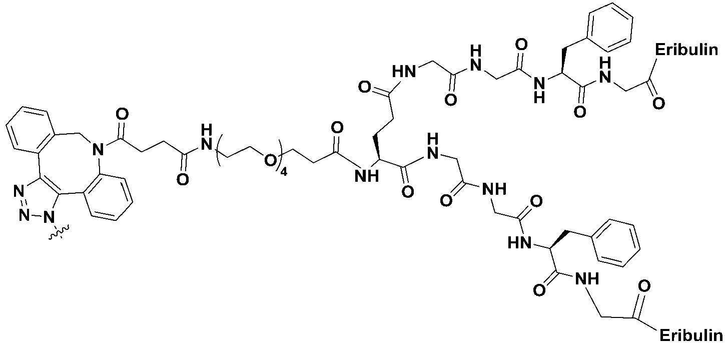 |
| ADC-4 | 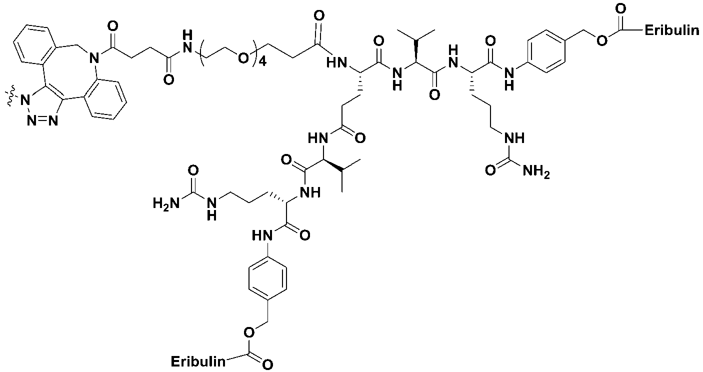 |
| ADC-6 | 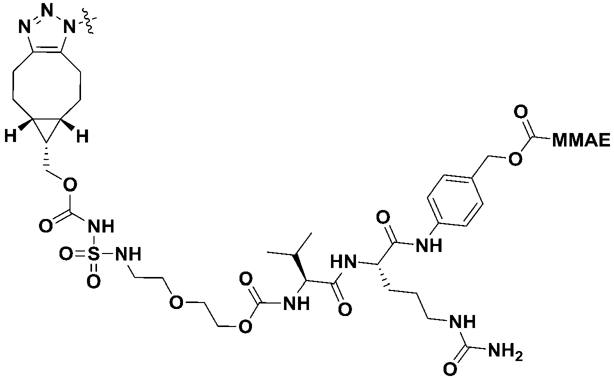 |

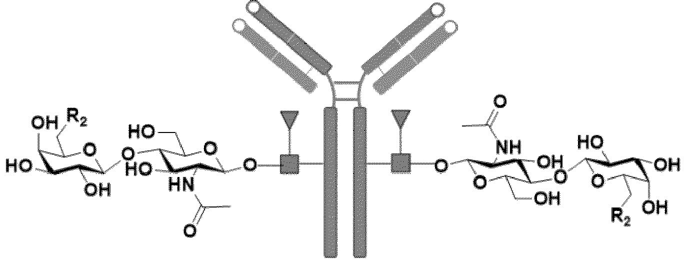

| ADC No. | R₂ |
|---|---|
| ADC-5 | 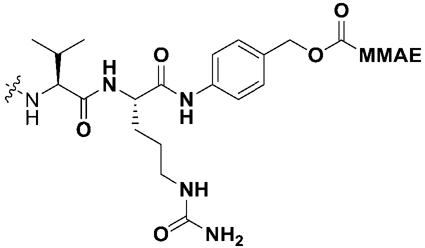 |

wherein in the above **ADC-1 to ADC-6,** the antibody is Sacituzumab;

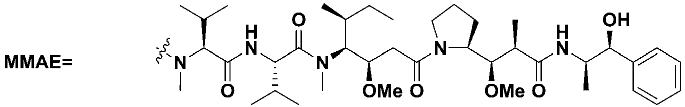

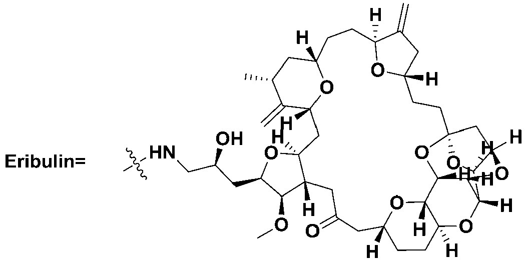

Specifically, the TROP2-targeting antibody-drug conjugate has the structure shown in ADC-5. In a second aspect, the present invention provides a method for preparing the TROP2-targeting antibody-drug conjugate **I-1** or **I-2** in the first aspect: 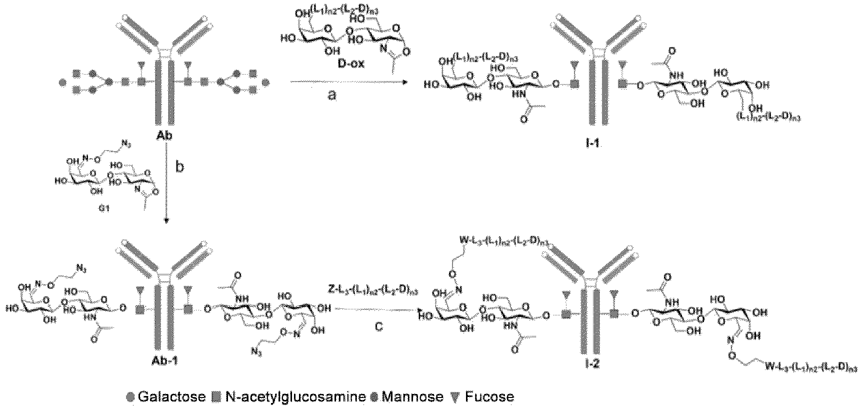
wherein Z represents an orthorhombic group capable of reacting with an azide group (N₃) to form the structure of W, such as a dibenzocyclooctyl (DBCO) group or a bicyclo[6.1.0]nonyne (BCN) group;
Ab, W, L₁, L₂, L₃, D, n2, and n3 are each defined as described above;

### Method 1: Preparation of antibody-drug conjugate I-1

Step a: reacting the toxin small molecule drug-disaccharide oxazoline complex D-ox with a wild-type antibody Ab in the present of the wild-type endoglycosidase Endo-S2 to obtain the antibody-drug conjugate **I-1** (**ADC-5**);

### Method 2: Preparation of Antibody-Drug Conjugate I-2

Step b: reacting the azide-disaccharide oxazoline **G1** with a wild-type antibody in the presence of the endoglycosidase Endo-S2 to obtain the non-natural glycoengineered antibody **Ab-1;**

Step c: reacting the non-natural glycoengineered antibody **Ab-1** with a toxin small molecule drug-linker Z-L₃-(L₁)ₙ₂-(L₂-D)ₙ₃ (i.e., compounds **D1, D4, D6**) containing an orthorhombic group that can react with the azide group (N₃) to obtain the antibody-drug conjugate **I-2** (**ADC-1** to **ADC-4** and **ADC-6**).

In some embodiments, in step a of the above method 1, the concentrations of the toxin small molecule drug-disaccharide oxazoline complex D-ox, the wild-type antibody, and the wild-type endoglycosidase Endo-S2 are 0.5 mM, 5 mg/mL, and 0.4 mg/mL, respectively; the reaction is carried out at 30 °C for 1 h, at pH 6.5.

In an embodiment, in method 1, the toxin small molecule drug-disaccharide oxazoline complex D-ox has the following structure:

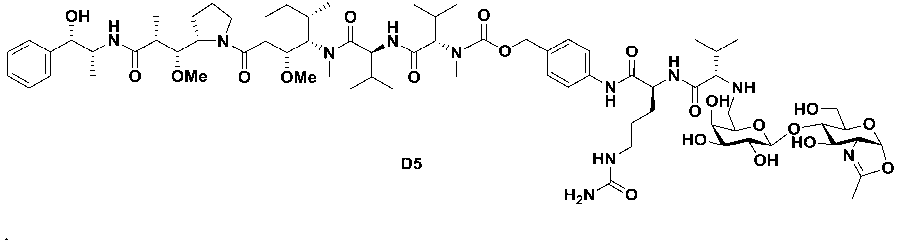

In step b of the above method 2, the concentrations of the azide-disaccharide oxazoline **G1,** the wild-type antibody, and the endoglycosidase Endo-S2 are 0.5 mM, 5 mg/mL, and 0.4 mg/mL, respectively; the reaction is carried out at 30 °C for 1 h, at pH 6.5.

In step c of the above method 2, the concentrations of the non-natural glycoengineered antibody **Ab-1** and the toxin small molecule drug-linker are 5 mg/mL and 0.3 mM, respectively; the reaction is carried out at 37 °C in a 50 mM PB buffer system at pH 7.4.

In some embodiments, in method 2, the toxin small molecule drug-linker Z-L₃-(L₁)ₙ₂-(L₂-D)ₙ₃ is selected from compounds with the following structures:

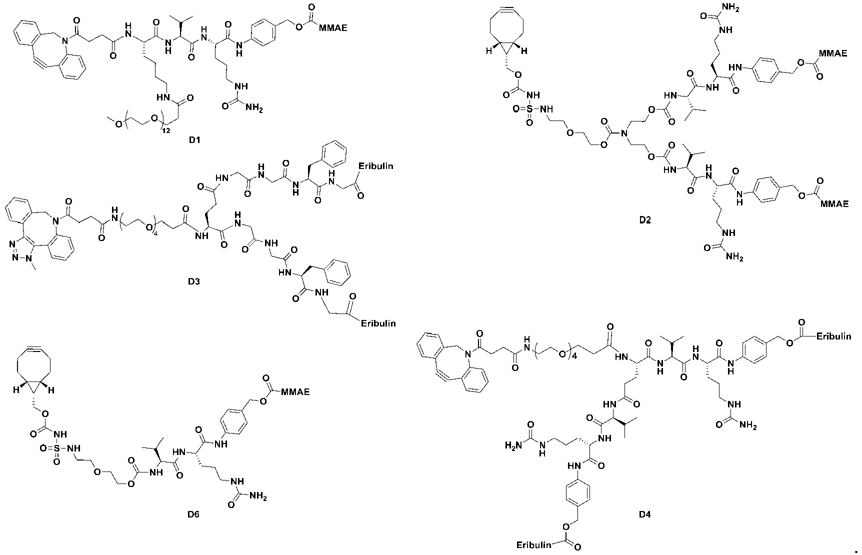

In a third aspect, the present invention provides use of the TROP2-targeting antibody-drug conjugates in the first aspect and the antibody-drug conjugates prepared by the method in the second aspect in the preparation of antitumor drugs.

In some embodiments, the tumor is a TROP2-highly expressing tumor. Preferably, the tumor includes: breast cancer (especially squamous cell carcinoma of the breast), gastric cancer, pancreatic cancer, colon cancer, bladder cancer, oral squamous cell carcinoma, ovarian cancer, urothelial carcinoma, lung cancer, especially breast cancer (especially squamous cell carcinoma of the breast) and gastric cancer.

### Beneficial Effects

The present invention utilizes a novel sugar site-specific conjugation technology to specifically link cytotoxins such as MMAE and Eribulin to antibody glycosylation sites. The obtained ADC compounds exhibited good in vitro homogeneity, stability, and toxicity against cells with high TROP2 expression. In particular, **ADC-1~2** and **ADC-5~6** showed almost no toxicity to cells with low TROP2 expression. More specifically, **ADC-5** also showed almost no toxicity to cells with moderate TROP2 expression. This selectivity of drug action can improve drug safety.

In the NCI-N87 tumor model, ADC-5 exhibited better in vivo tumor-suppressive activity than other glycan site-specific ADC compounds (such as DS1062 and **ADC-7**).

### BRIEF DESCRIPTION OF THE DRAWINGS

Figure 1 shows the hydrophobic interaction chromatography (HIC) analysis data of the antibody-drug conjugate (ADC) in Test Example 1.
Figure 2 shows the size exclusion chromatography (SEC) analysis data of the antibody-drug conjugate (ADC) in Test Example 2.
Figure 3 shows the reverse phase chromatography (RP) analysis data of the antibody-drug conjugate (ADC) in Test Example 3.
Figure 4 shows the size exclusion chromatography (SEC) analysis data of the aggregation stability test of the antibody-drug conjugate (ADC) in Test Example 4.
Figure 5 shows the in vitro activity test results of the antibody-drug conjugate (ADC) in Pharmacological Example 1.
Figure 6 shows the in vivo activity test results of the antibody-drug conjugates ADC-5 and ADC-7 in NCI-N87 tumor-transplanted mice in Pharmacological Example 2.

### DETAILED EMBODIMENTS

The endoglycosidases used in the present invention were expressed in the *E. coli* system in the laboratory. The small molecule cytotoxic drugs Eribulin and monomethyl aurestatin E (MMAE) were purchased from Shanghai Haoyuan Chemexpress Co., Ltd.; DBCO compounds (e.g., compound 5) and BCN compounds (e.g., compounds 7 and 25) were purchased from Chengdu Baierkang Biotechnology Co., Ltd.; 3-azidopropylamine was purchased from Shanghai Bide Pharmatech Co. Ltd. K₂HPO₄, (NH₄)₂SO₄, and KH₂PO₄ were purchased from Bide Pharmatech Co. Ltd; 20×PBS was purchased from Sinopharm Group. Other compounds and reagents, unless otherwise specified, were purchased from Sinopharm Chemical Reagent Co., Ltd.

The instruments used in the present invention include: a high-resolution mass spectrometer (Waters Xevo G2-XS QTOF), an analytical high-performance liquid chromatograph (Thermo Ultimate 3000), a high-performance liquid chromatograph (WATERS W2690), a high-performance liquid chromatograph (Angilent 1260), an analytical high-performance liquid chromatograph (Beijing Tong Heng Innovation Technology Co., Ltd, LC3000), and a preparative high-performance liquid chromatograph (Beijing Tong Heng Innovation Technology Co., Ltd, LC3000). The chromatographic columns include: Thermo C18 (Acclaim^{™} 120, 5 µm, 4.6 x 250 mm), Agilent SB C18 (5 µm, 4.6 x 150 mm), and Waters C18 columns (ACQUITY UPLC BEH C18, 1.7 µm, 2.1 x 50 mm). Waters C4 column (ACQUITY UPLC Protein BEH C4, 1.7 µm, 2.1 mm x 50 mm). Thermo, MAbPac HIC-Butyl column (4.6 x 100 mm, 3.5 µm). Agilent PLRP-S 1000Å column (4.6 x 250 mm, 8 µm). BioCore SEC-300 column (5 µm, 7.8 x 300 mm).

### I: Synthesis of azide-disaccharide oxazoline G1

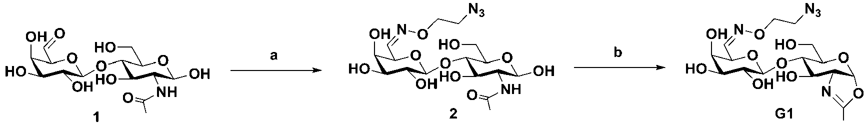

Step 1: 18 mg (47.2 µmol) of CHO-LacNAc 1 was weighed and dissolved in 200 µL of a 50 mM pH 7.4 PB buffer. 5.3 mg (51.9 µmol) of O-(2-azidoethyl)hydroxylamine hydrochloride was added to the above reaction system and reacted at 37 °C for 2 h. LC-MS monitoring showed the reaction was completed. After purification using a semi-preparative C18 column, compound 2 (20 mg, 91% yield) was obtained. HRMS calculated for C₁₆H₂₇N₃O₁₁ [M+H]⁺: 466.1785, found: 466.1732.

Step 2: compound 2 (10 mg, 21.4 µmol) was weighed and dissolved in 428 µL of D₂O. 2-chloro-1,3-dimethyl-1H-benzimidazole-3-chloride (CDMBI, 23.1 mg, 107 µmol) was added to the above system. After mixing thoroughly, the system was cooled to 0 °C on ice, added with potassium phosphate (68.5 mg, 321 µmol) and reacted at 0 °C for 12 h. LC-MS monitoring showed the reaction was completed. The resultant was separated and purified using a semi-preparative C18 column, and the target product was collected and lyophilized to obtain compound **G1**. HRMS calculated for C₁₆H₂₅N₅O₁₀ [M+H]⁺: 448.1679, found: 448.1662.

### II: Synthesis of Non-Natural Glycoengineered Antibodies

Non-natural glycoengineered antibody **Ab-1** was obtained by reacting compound **G1** with the wild-type Sacituzumab antibody via step b of the General Procedure II for preparing ADCs.

The preparation method for non-natural glycoengineered antibody **Ab-2** was as follows:

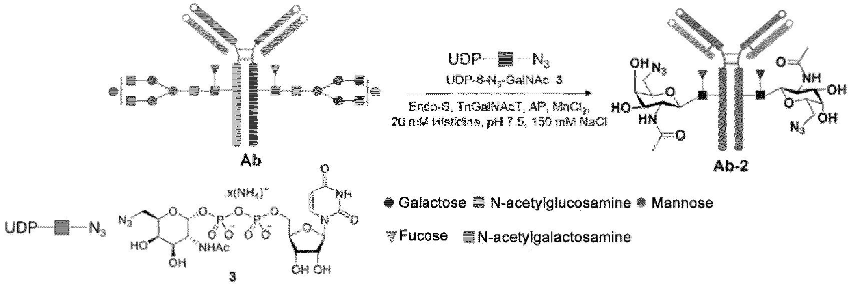

The wild-type Sacituzumab antibody, endoglycosidase Endo-S, phospholipase (AP), UDP-6-N3-GalNAc 3, and TnGalNAcT (purchased from Wuhan Tangzhi Pharmaceutical Co., Ltd.) were added sequentially to a buffer solution (20 mM histidine, 150 mM NaCl, pH 6.5) at reaction concentrations of 7 mg/mL, 0.08 mg/mL, 0.75 µg/mL, 0.5 mM, and 0.2 mg/mL, respectively. After adjusting the pH of the reaction system to 7.5, 10% of 10 mM MnCl₂ was added to the system, and the mixture was incubated at 30 °C for 16 hours. LC-MS confirmed a product with two azide groups was formed, and the product was purified using Protein A to obtain the desired glycoengineered antibody **Ab-2.**

### III: Preparation of Small Molecule Drug-Linker

### Example 1: Synthesis of Compound D1

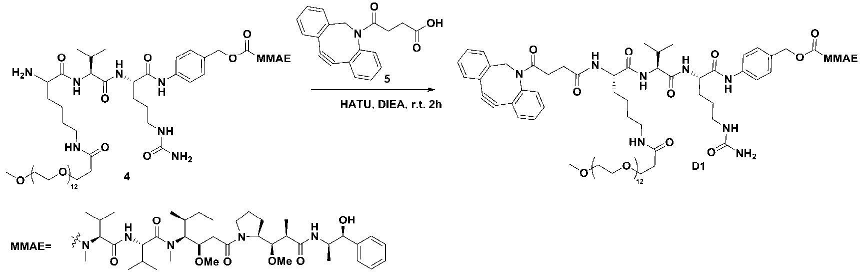

### Procedure : Compound 5 (8.06 mg, 0.0264 mM) and

2-(7-azobenzotriazole)-N,N,N',N'-tetramethyluronium hexafluorophosphate (HATU, 16.73 mg, 0.044 mM) were weighed and dissolved in 650 µL of N,N-dimethylformamide (DMF). Compound **4** (41 mg, 0.022 mM) and N,N-diisopropylethylamine (DIEA, 11.5 µL, 0.066 mM) were added sequentially to the above system. After mixing the reaction system, the reaction was carried out at room temperature for 2 hours. LC-MS monitoring showed the reaction was complete. The resultant was purified by using a semi-preparative C18 column, and the target product was collected and lyophilized to obtain compound **D1** (21.7 mg, yield 45.8%). HRMS: [M+H]⁺ 2153.283.

### Example 2: Synthesis of compound D2

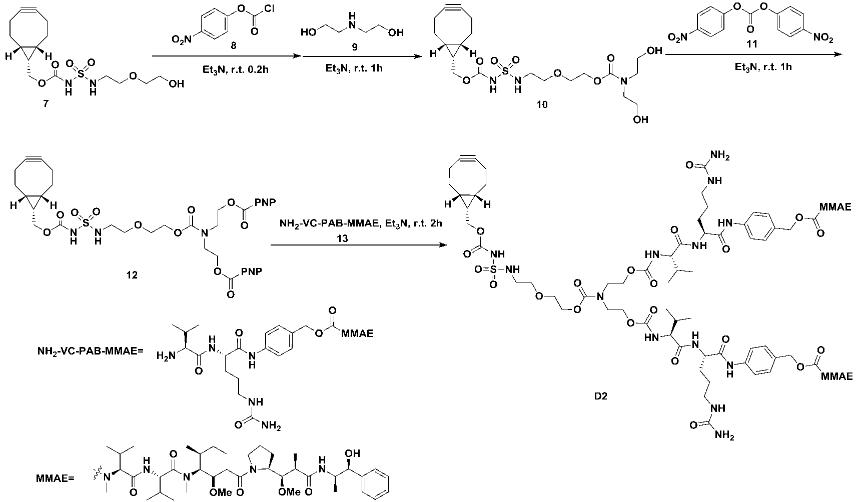

Step 1: compound **7** (31 mg, 86 µM) was weighed and dissolved in 0.4 mL of N,N-dimethylformamide (DMF). Compound **8** (36 mg, 18 µM) and triethylamine (20 µL, 14.2 µM) were added sequentially to the above system, and reacted at room temperature for 20 minutes. The reaction system was used directly for the next step without purification.

Step 2: compound **9** (75 µL, 20.5 µM) and triethylamine (20 µL, 14.2 µM) were added sequentially to the above crude product (31 mg, 32.4 µM), and reactedfor 1 hour. Compound **10** was obtained as monitored by LC-MS. The reaction system was used directly for the next step without purification.

Step 3: Compound **11** (62.4 mg, 32.4 µM) and triethylamine (20 µL, 14.2 µM) were added to the above crude product, and reacted for 1 hour. After the reaction was complete as monitored by LC-MS, the product was purified by a semi-preparative C18 column and then lyophilized to obtain compound **12,** with a yield of 33.3%.

Step 4: Compound **12** (16 mg, 19.48 µM) was weighed and dissolved in 1 mL of N,N-dimethylformamide (DMF). Compound **13** (54.7 mg, 48.7 µM) and triethylamine (10.8 µL, 7.92 µM) were added sequentially to the above system, and reacted at 37 °C for 2 hours. After the reaction was complete as monitored by LC-MS, the product was purified by reversed-phase chromatography, and the target fraction was collected and lyophilized to obtain compound **D2** as a white solid. HRMS: [M+H]⁺ 2788.5628.

### Example 3: Synthesis of Compound D3

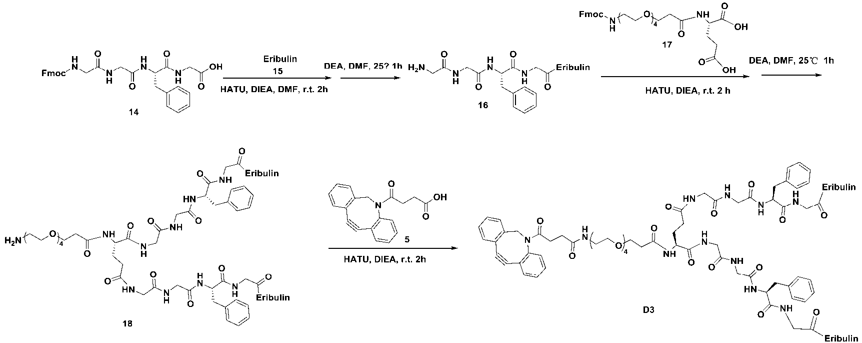

### Step 1: compound 14 (11.48 mg, 0.0205 mM) and

2-(7-azobenzotriazole)-N,N,N',N'-tetramethyluronium hexafluorophosphate (HATU, 11.67 mg, 0.0307 mM) were weighed and dissolved in 2 mL of N,N-dimethylformamide (DMF). Compound **15** (15 mg, 0.0205 mM) and N,N-diisopropylethylamine (DIEA, 7.94 mg, 0.0614 mM) were added sequentially to the above system. The reaction system was mixed and then reacted at room temperature for 2 hours. LC-MS showed that there was virtually no reactant remaining, and the main peak was the product. The reaction solution was directly used for the next chemical reaction. HRMS: [M+H-18]⁺ 1271.8.

Step 2: Diethylamine (DEA, 14.99 mg, 0.205 mM) was added dropwise to the reaction solution from the previous step, and the mixture was stirred at room temperature for 1 hour. LC-MS showed that the starting material had reacted completely and a product had formed. After centrifugation, the reaction solution was subjected to semi-preparative separation and purification. The target product was collected and lyophilized to obtain compound **16** (14 mg, yield 65.15%), HRMS: [M-H]⁺ 1048.4.

### Step 3: compound 17 (4.12 mg, 0.0067 mM) and

2-(7-azobenzotriazole)-N,N,N',N'-tetramethyluronium hexafluorophosphate (HATU, 7.64 mg, 0.0201 mM) were weighted and dissolved in 2 mL of N,N-dimethylformamide (DMF). Compound 16 (14 mg, 0.0134 mM) and N,N-diisopropylethylamine (DIEA, 5.20 mg, 0.0402 mM) were added sequentially to the above system. The reaction system was mixed and then reacted at room temperature for 2 hours. LC-MS showed that there was virtually no reactant remaining, and the main peak was the product. The reaction solution was directly used for the next chemical reaction. HRMS: [M/2+H]⁺ 1339.2.

Step 4: diethylamine (DEA, 9.93 mg, 0.134 mM) was added dropwise to the reaction solution from the previous step, and stirred at 25 °C for 1 hour. LCMS showed that the starting material had reacted completely and a product had formed. After centrifugation, the reaction solution was subjected to semi-preparative separation and purification. The target product was collected and lyophilized to obtain compound **18** (8 mg, yield 24.32%), HRMS: [M/2+H]⁺ 1228.2.

### Step 5: compound 5 (2.0 mg, 0.0066 mM) and

2-(7-azobenzotriazole)-N,N,N',N'-tetramethyluronium hexafluorophosphate (HATU, 1.88 mg, 0.0049 mM) were weighted and dissolved in 2 mL of N,N-dimethylformamide (DMF). Compound **18** (8.0 mg, 0.0033 mM) and N,N-diisopropylethylamine (DIEA, 1.28 mg, 0.0099 mM) were added sequentially to the above system. The reaction system was mixed and then reacted at room temperature for 2 hours. LC-MS showed that the starting materials had reacted completely and products had formed. After centrifugation, the reaction solution was subjected to semi-preparative separation and purification. The target product was collected and lyophilized to obtain compound **D3** (5.2 mg, yield 40.32%). HRMS: [M+H]⁺ 2742.19.

### Example 4: Synthesis of Compound D4

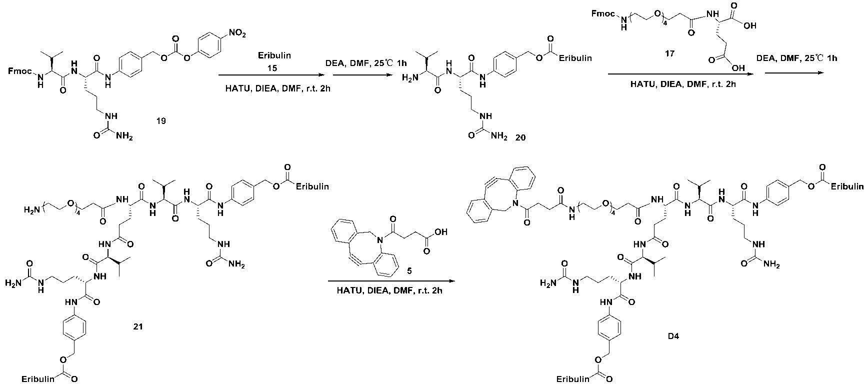

### Step 1: compound 19 (15.72 mg, 0.0205 mM) and

2-(7-azobenzotriazole)-N,N,N',N'-tetramethyluronium hexafluorophosphate (HATU, 11.67 mg, 0.0307 mM) were weighed and dissolved in 2 mL of N,N-dimethylformamide (DMF). Compound **15** (15 mg, 0.0205 mM) and N,N-diisopropylethylamine (DIEA, 7.95 mg, 0.0615 mM) were added sequentially to the above system. The reaction system was mixed and then reacted at room temperature for 2 hours. LC-MS showed that there was virtually no reactant remaining, and the main peak was the product. The reaction solution was directly used for the next chemical reaction. HRMS: [M+H]⁺ 1357.8.

Step 2: Diethylamine (DEA, 14.99 mg, 0.205 mM) was added dropwise to the reaction solution from the previous step, and the mixture was stirred at 25 °C for 1 hour. LC-MS showed that the starting material had reacted completely and a product had formed. After centrifugation, the reaction solution was subjected to semi-preparative separation and purification. The target product was collected and lyophilized to obtain compound **20** (18 mg, yield 77.34%). HRMS: [M-H]⁺ 1135.8.

### Step 3: compound 17 (3.26 mg, 0.00528 mM) and

2-(7-azobenzotriazole)-N,N,N',N'-tetramethyluronium hexafluorophosphate (HATU, 6.02 mg, 0.0158 mM) were weighed and dissolved in 2 mL of N,N-dimethylformamide (DMF). Compound **20** (12 mg, 0.0105 mM) and N,N-diisopropylethylamine (DIEA, 4.09 mg, 0.0317 mM) were added sequentially to the above system. The reaction system was mixed and then reacted at room temperature for 2 hours. LC-MS showed that there was virtually no reactant remaining, and the main peak was the product. The reaction solution was directly used for the next chemical reaction. HRMS: [M+H]⁺ 1426.3.8.

Step 4: diethylamine (DEA, 7.78 mg, 0.105 mM) was added dropwise to the reaction solution from the previous step, and stirred at 25 °C for 1 hour. LC-MS showed that the starting material had reacted completely and a product was formed. After centrifugation, the reaction solution was subjected to semi-preparative separation and purification. The target product was collected and lyophilized to obtain compound **21** (5.5 mg, 19.92%). HRMS: [M/2+H]⁺ 1315.1.

### Step 5: compound 5 (1.28 mg, 0.0042 mM) and

2-(7-azobenzotriazole)-N,N,N',N'-tetramethyluronium hexafluorophosphate (HATU, 1.20 mg, 0.00315 mM) were weighed and dissolved in 2 mL of N,N-dimethylformamide (DMF). Compound **21** (5.5 mg, 0.0021 mM) and N,N-diisopropylethylamine (DIEA, 0.81 mg, 0.0063 mM) were added sequentially to the above system. The reaction system was mixed and then reacted at room temperature for 2 hours. LC-MS showed that the starting material had reacted completely and a product was formed. After centrifugation, the reaction solution was subjected to semi-preparative separation and purification. The target product was collected and lyophilized to obtain compound **D4** (1.7 mg, yield 27.76%). HRMS: [M+H]⁺ 1459.7.

### Example 5: Synthesis of Compound D5

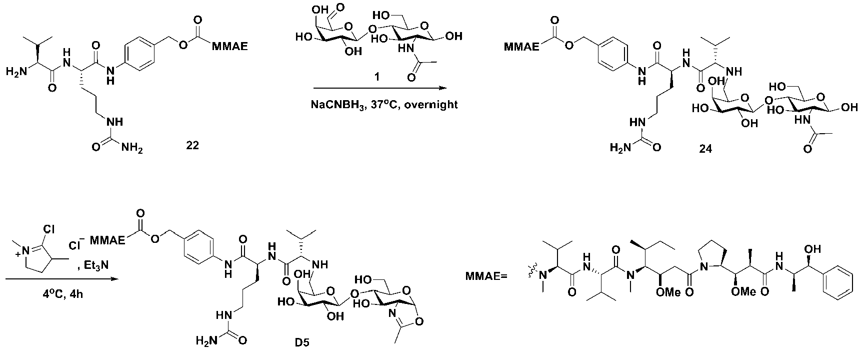

Step 1: compound **22** (20 mg, 0.0178 mM) was weighed and dissolved in 200 µL of N,N-dimethylformamide (DMF). Compound **1** (27 mg, 0.0712 mM) and sodium cyanoborohydride (NaCNBH₃, 22.4 mg, 0.356 mM) were added sequentially to the above system. The reaction system was mixed and then reacted overnight at 37 °C. LC-MS showed that most of the starting material had converted to the product. The product was purified using a semi-preparative C18 column, and the target product was collected and lyophilized to obtain product **24** (15 mg, yield 56.6%). HRMS: [M+H] ⁺ 1488.8453.

Step 2: Compound 24 was weighed and dissolved in deionized water (ddH₂O), and added sequentially with 2-1,3-dimethylimidazolium chloride (DMC) and triethylamine, and their final concentrations was 10 mM, 200 mM, and 600 mM, respectively. The reaction system was mixed and reacted at 4 °C for 4 h. LC-MS monitoring showed that the reaction was almost complete. The product was purified using an alkaline semi-preparative C18 column, and the target product was collected, and lyophilized to obtain compound **D5.** HRMS: [M+2H]²⁺ 735.9212.

### Example 6: Synthesis of Compound D6

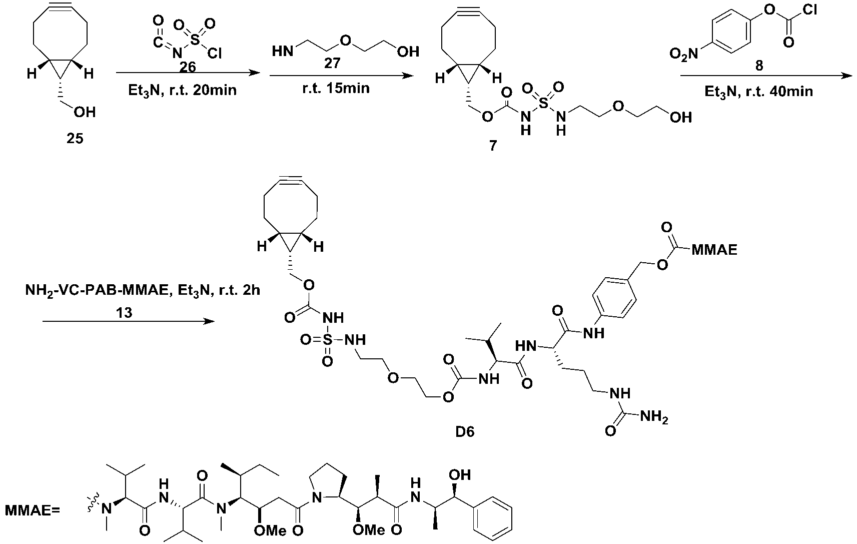

Step 1: compound **25** (50 mg, 33 µM) was weighed and dissolved in 4 mL of dichloromethane. Compound **26** (29 µL, 20.4 µM) and triethylamine (40 µL, 27.2 µM) were added sequentially to the reaction system. After reacting at room temperature for 20 minutes, compound **27** (94 µL, 71 µM) was added to the reaction system and the reaction was carried out for 15 minutes. After the reaction was complete as monitored by LC-MS, the resultant was extracted with 150 µL of dichloromethane and blow-dried to obtain crude compound **7.**

Step 2: crude compound **7** (31 mg, 86 µM) from the previous step was weighed and dissolved in 1 mL of N,N-dimethylformamide (DMF). Compound 8 (36 mg, 18 µM) and triethylamine (20 µL, 14.2 µM) were added thereto. The reaction was carried out at room temperature for 40 minutes, and the reaction was complete as monitored by LC-MS. The product was used directly for the next step.

Step 3: Compound **13** (54.7 mg, 48.7 µM) was added to the crude compound from the previous step (25.2 mg, 48 µM), followed by triethylamine (62.5 µL, 44.5 µM). The reaction was carried out at room temperature for 2 hours. The reaction was complete as monitored by LC-MS. The product was purified using a semi-preparative C18 column and lyophilized to obtain compound **D6** (yield 61.3%). HRMS: [M+H]+ 1508.82.

### IV: Synthesis of Glycan Site-Specific Antibody-Drug Conjugates ADC-1 to ADC-7

The preparation route of the glycan site-specific antibody-drug conjugates (ADCs) of the present invention is summarized in the following reaction schemes.

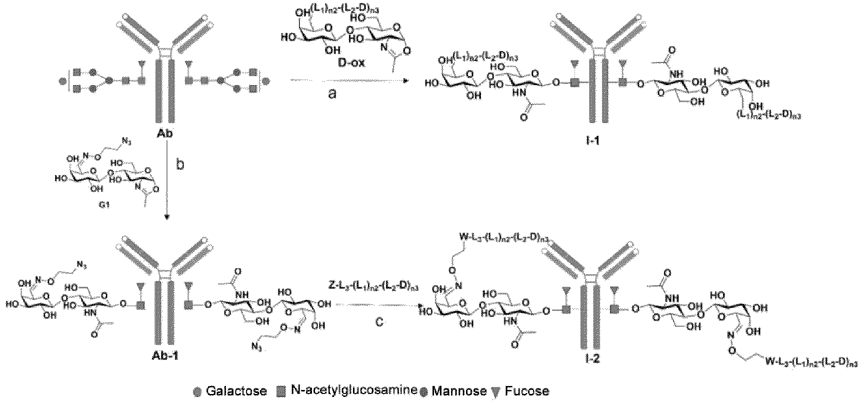

### General Procedure I for ADC Preparation:

Step a: The prepared toxin-disaccharide oxazoline complex D-ox (e.g., compound **D5**), the wild-type Sacituzumab antibody, and the wild-type endoglycosidase Endo-S2 were added to a 50 mM PB at concentrations of 0.5 mM, 5 mg/mL, and 0.4 mg/mL, respectively. The pH of the reaction system was adjusted to 6.5, and the mixture was incubated at 30 °C for 1 h. LC-MS confirmed the formation of a product containing two disaccharide-toxin complexes, and the product was purified by protein A to obtain the target antibody-drug conjugate **I-1** (e.g., **ADC 5**).

### General Procedure II for ADC Preparation:

Step b: The prepared azide-disaccharide oxazoline G1, the wild-type Sacituzumab antibody, and the endoglycosidase Endo-S2 were added to a 50 mM PB at concentrations of 0.5 mM, 5 mg/mL, and 0.4 mg/mL, respectively. The pH of the reaction system was adjusted to 6.5, and the mixture was incubated at 30 °C for 1 h and the resultant was purified by Protein A to obtain the desired non-natural glycoengineered antibody **Ab-1.**

Step c: the prepared non-natural glycoengineered antibody **Ab-1** and a drug-linker Z-L₃-(L₁)ₙ₂-(L₂-D)ₙ₃ (e.g., compounds **D1, D4, D6**) were added to a 50 mM pH 7.4 PB buffer system containing no more than 20% DMF at concentrations of 5 mg/mL and 0.3 mM, respectively. After confirming pH of the final reaction system was 7.4, the system was incubated at 37 °C for 2 hours. After the conversion was complete as confirmed by LC-MS, the product was purified by protein A to obtain the desired antibody-drug conjugate **I-2** (e.g., **ADC-1 to ADC-4** and **ADC-6**).

Note: Several factors need to be considered when choosing General Procedure I or II to prepare ADC compounds: (1) endoglycosidases have different enzyme recognition abilities for different substrates. A molecule with a large structure has a weak enzyme recognition ability, and thus General Procedure II should be adopted; (2) endoglycosidases specifically recognize glycooxazoline structures, which needs to be prepared under alkaline conditions. Therefore, General Procedure II should be adopted for those drug-linkers intolerant to alkaline conditions.

### Example 7: Synthesis of ADC-1~ADC-4 and ADC-6

Antibody-drug conjugates **ADC-1~ADC-4** and **ADC-6** were obtained from **compounds D1~ D4** and **D6,** and the non-natural glycoengineered antibody **Ab-1** by using the method in step c of General Procedure II for ADC Preparation, respectively. Their structures are as follows:

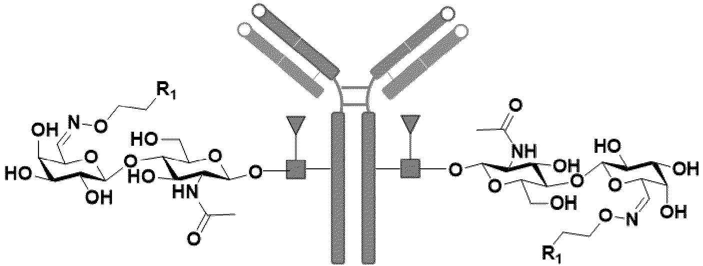

| ADC No. | R₁ |
|---|---|
| **ADC-1** | 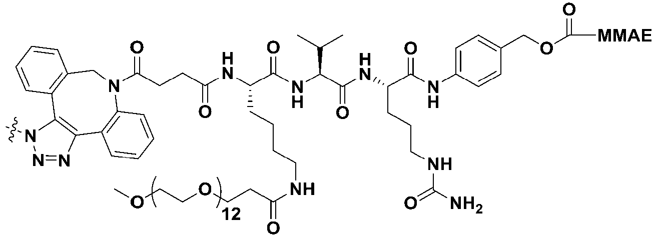 |
| **ADC-2** | 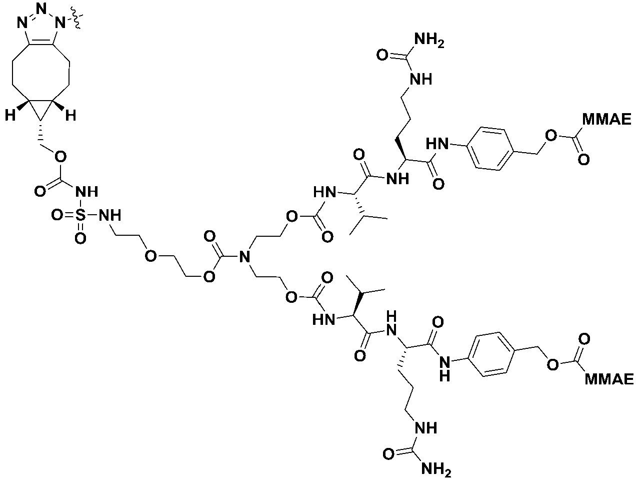 |
| **ADC-3** | 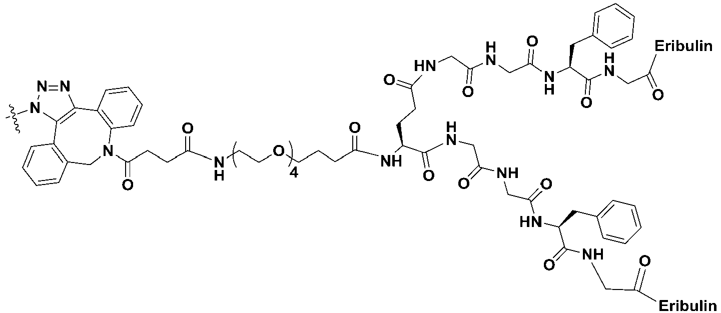 |
| **ADC-4** | 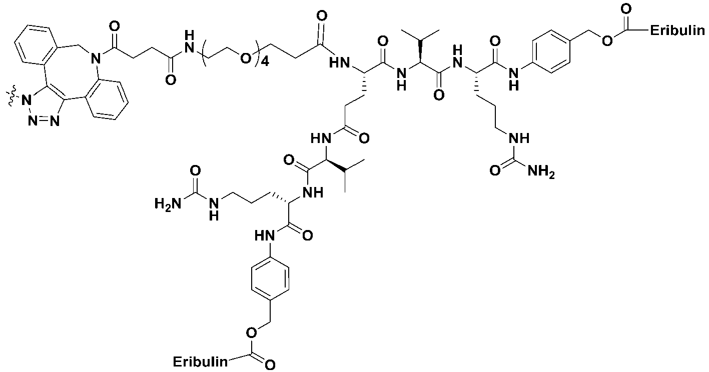 |
| **ADC-6** | 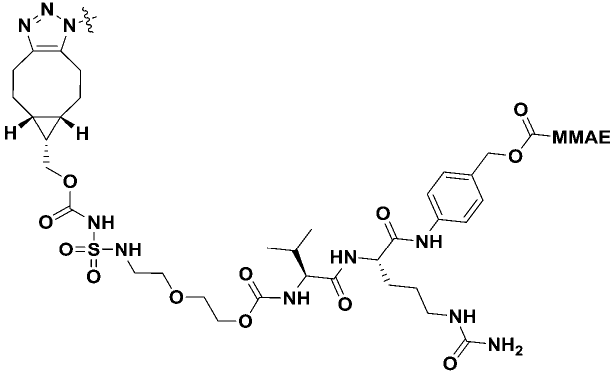 |

Wherein,

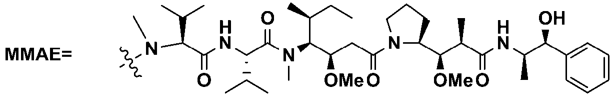

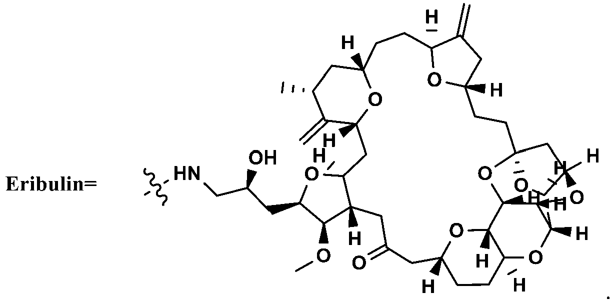

### Example 8: Synthesis of ADC-5

The antibody-drug conjugate **ADC-5** was obtained from the compound **D5** and the wild-type Sacituzumab antibody by the method in general procedure I for ADC preparation. Its structure is as follows:

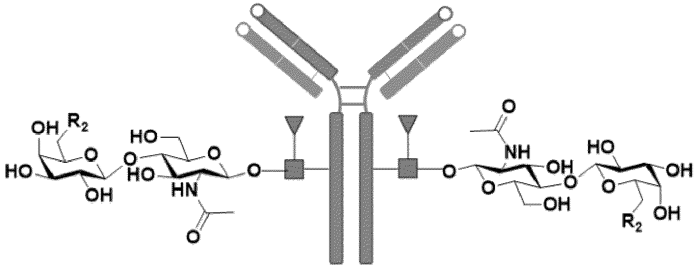

| ADC No. | R₂ |
|---|---|
| ADC-5 | 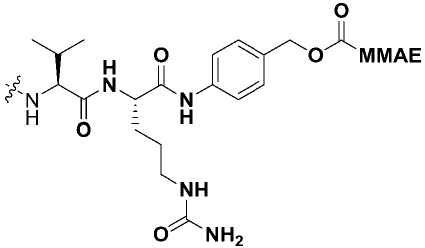 |

### Comparative Example 1: Synthesis of ADC-7

The non-natural glycoengineered antibody **Ab-2** prepared above and the drug-linker (i.e., compound **D6**) were added to a 50 mM pH 7.4 PB buffer system at concentrations of 5 mg/mL and 0.3 mM respectively. After confirming that the final reaction system pH was 7.4, the mixture was incubated at 37 °C. After conversion was complete as confirmed by LC-MS, the product was purified by protein A to obtain the desired antibody-drug conjugate **ADC-7.**

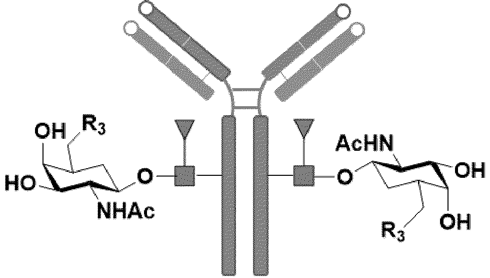

| ADC No. | R₃ |
|---|---|
| ADC-7 | 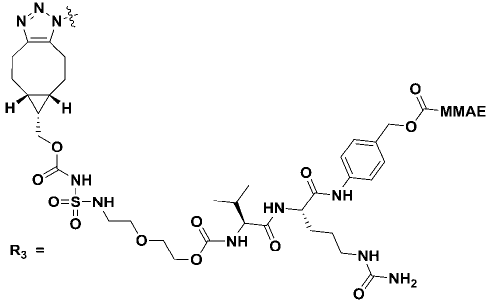 |

**Test Example 1:** Chromatographic Detection of Hydrophobic Interactions of ADC Compounds In this test example, the chromatographic detection of hydrophobic interactions of ADC compounds was carried out under the following conditions:
Sample Preparation: the ADC samples were diluted to 1 mg/mL with 1 x PBS.

Mobile Phase Preparation: phase A: 88.3 g ammonium sulfate and 7.878 g sodium phosphate were brought to the volume 0.95 L with pure water, adjusted to pH 7.0, and added with 50 mL isopropanol. Phase B: 6.56 g sodium phosphate was brought to the volume 0.8 L with pure water, adjusted the pH to 7.0, and added with 200 mL isopropanol. They were sterilized by filtering through a 0.22 µm PES membrane, and sonicated for 20 min for later use.

### Chromatographic Conditions:

| | Parameter settings |
|---|---|
| Column | Thermo, MAbPac HIC-Butyl, 5 µm, 4.6*100mm |
| Mobile phase | Phase A -phase B |
| Injection concentration | 1 mg/mL |
| Injection volume | 20 L |
| Column Temperature | Room temperature |
| Flow rate | 0.6 mL/min |
| UV detector wavelength | 280 nm |

The mobile phase gradient was set as shown in the table below:

| t (min) | Flow rate (mL/min) | A% | B% |
|---|---|---|---|
| 0 | 0.6 | 70 | 30 |
| 15 | 0.6 | 0 | 100 |
| 20 | 0.6 | 0 | 100 |
| 20.1 | 0.6 | 70 | 30 |
| 26 | 0.6 | 70 | 30 |

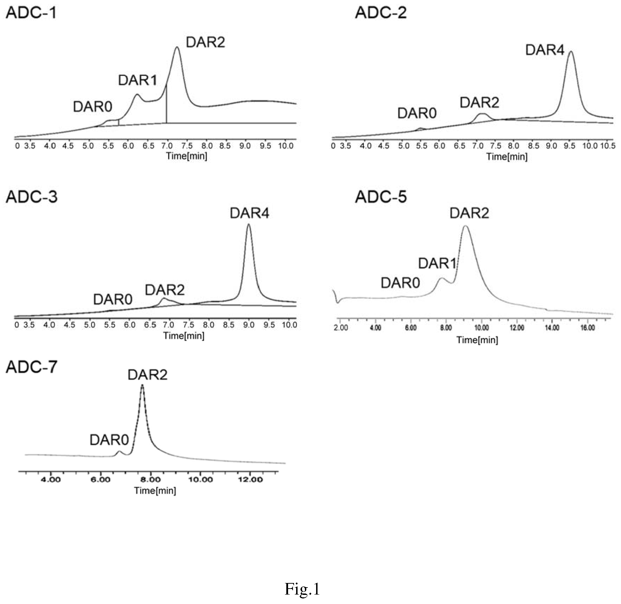

The hydrophobic interaction chromatograms of the ADCs are shown in Figure 1, indicating that the ADCs prepared in the present invention have good drug-to-antibody ratio (DAR), and most ADCs exhibit good homogeneity.

### Test Example 2: Size Exclusion Chromatography (SEC) Analysis of ADCs

This test example performed SEC analysis of the ADC compounds under the following conditions:
Sample Preparation: The ADCs to be tested were diluted with 1 × PBS to a concentration of 1 mg/mL.

### Chromatographic Conditions:

| | Parameter settings |
|---|---|
| Column | BioCore SEC-300, 5 µm, 7.8*300mm |
| Mobile phase | 1 × PBS, Isocratic elution, elution time: 20 min |
| Injection volume | 20 µL |
| Column Temperature | Room temperature |
| Flow rate | 1 mL/min |

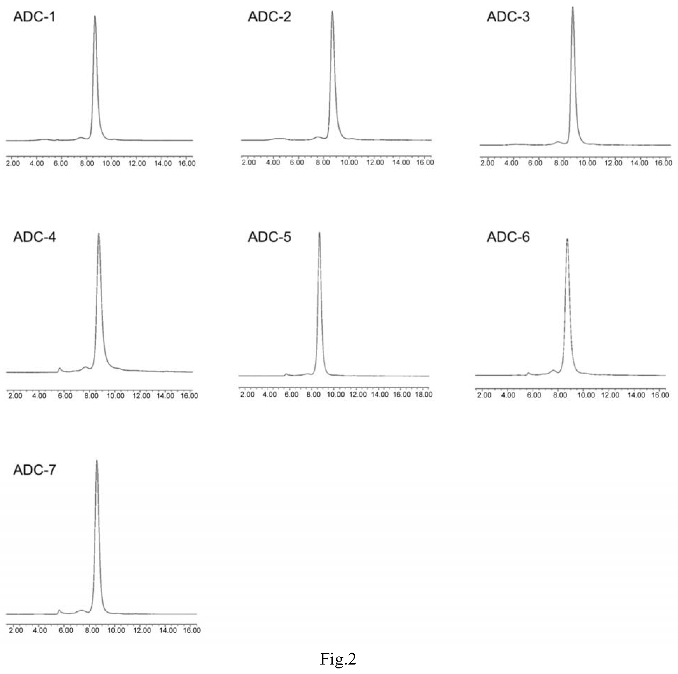

Size exclusion chromatograms (SEC) of the ADCs are shown in Figure 2, indicating that the ADCs prepared in the present invention have good purity.

**Test Example 3:** Reverse Phase Chromatography (RP) Analysis of ADCs This test example performed RP analysis of the ADC compound under the following conditions: Sample Preparation: The ADCs to be tested were diluted with 1 × PBS to a concentration of 1 mg/mL.

### Chromatographic Conditions:

| | Parameter settings |
|---|---|
| Column | Agilent, PLRP-S 1000Å, 4.6 x 250 mm, 8 µm |
| Mobile phase | A: water (containing 0.1 % TFA) ; B: acetonitrile (containing 0.1% TFA) |
| Injection concentration | 1 mg/mL |
| Injection volume | 25 µL |
| Column Temperature | 80 °C |
| Flow rate | 0.8 mL/min |

The mobile phase gradient was set as follows:

| t (min) | A% | B% |
|---|---|---|
| 0 | 30 | 70 |
| 20 | 10 | 90 |
| 21 | 10 | 90 |
| 26 | 30 | 70 |

**Table 1: DAR values of ADCs calculated based on the RP results.**

| Sample Name | L0 | H0 | H1 | H2 | DAR |
|---|---|---|---|---|---|
| ADC-1 | 100% | 12.36% | 87.63% | | 1.75 |
| ADC-2 | 100% | 25.64% | 12.82% | 61.52% | 2.72 |
| ADC-3 | 100% | 18.59% | 3.35% | 78.04% | 3.19 |
| ADC-4 | 100% | 14.83% | 4.92% | 80.23% | 3.31 |
| ADC-5 | 100% | 9.81% | 90.19% | | 1.80 |
| ADC-6 | 100% | 29.43% | 70.57% | | 1.41 |

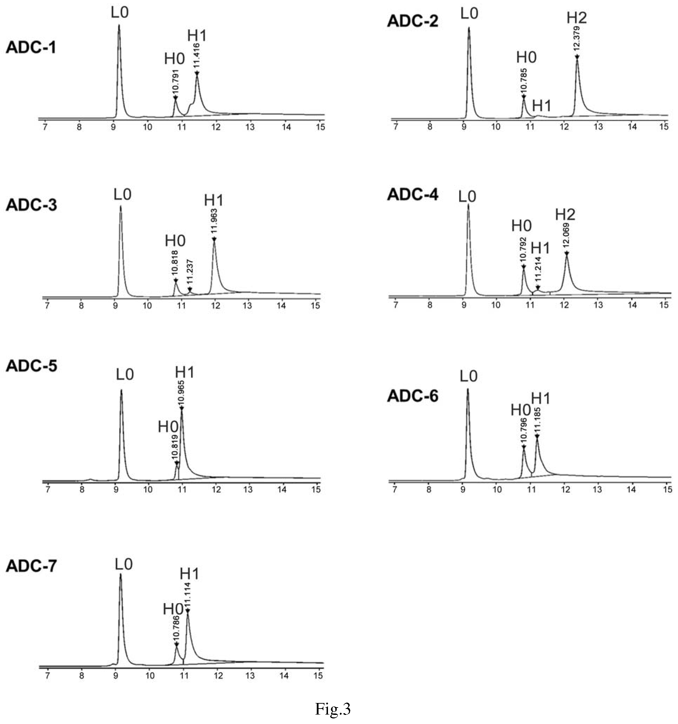

The RP analysis data of the ADC are shown in Figure 3 and Table 1, indicating that the ADCs prepared in the present invention have good drug-to-antibody ratios (DAR), which are comparable to the theoretical DAR value.

### Test Example 4: Aggregation Stability Analysis of ADCs

The prepared ADC compounds **ADC-1~2** and **ADC5~6** were heated at 60 °C, and samples were taken at 6 hours and 24 hours respectively to observe their aggregation. The samples were then analyzed using the size exclusion chromatography (SEC) method for ADCs in Test Example 2 above.

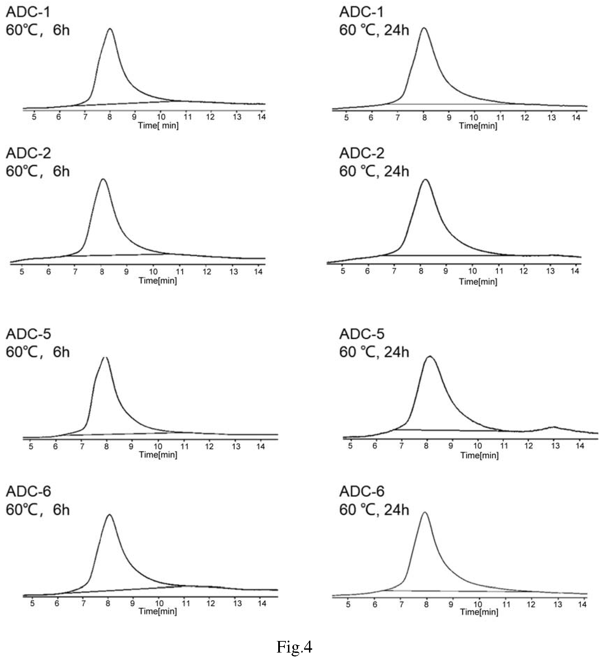

The observation results show that none of **ADC-1~2** and **ADC-5~6** exhibited aggregation. The SEC chromatograms are shown in Figure 4, indicating that **ADC-1~2** and **ADC-5~6** have good aggregation stability.

### Pharmacological Example 1: In Vitro Activity Assay of ADCs

TROP2 high-expressing cell lines BXPC-3, NCI-N87, MDA-MB-468 and SK-BR-3, TROP2 moderately-expressing cell line COLO205, and TROP2 low-expressing cell line MDA-MB-231 were all purchased from the Cell Bank of the Chinese Academy of Sciences (Shanghai). The cellular activity and cytotoxicity of the ADC molecules (**ADC-1~ADC-7**) prepared above were tested using MTT assay.

Specific procedures: Cells to be tested were seeded at 6000 cells/well in 96-well cell culture plates and cultured overnight. The drug was diluted 5-fold starting from the highest concentration of 100 nM, with 9 dilutions. The drug was added sequentially, with each dilution sample set up in triplicate. The outermost wells were added with PBS; wells B11, C11, and D11 were negative controls (wells without drug); wells E11, F11, and G11 were blank controls (wells without both cells and drug); ADC-7 was set up as a positive control.

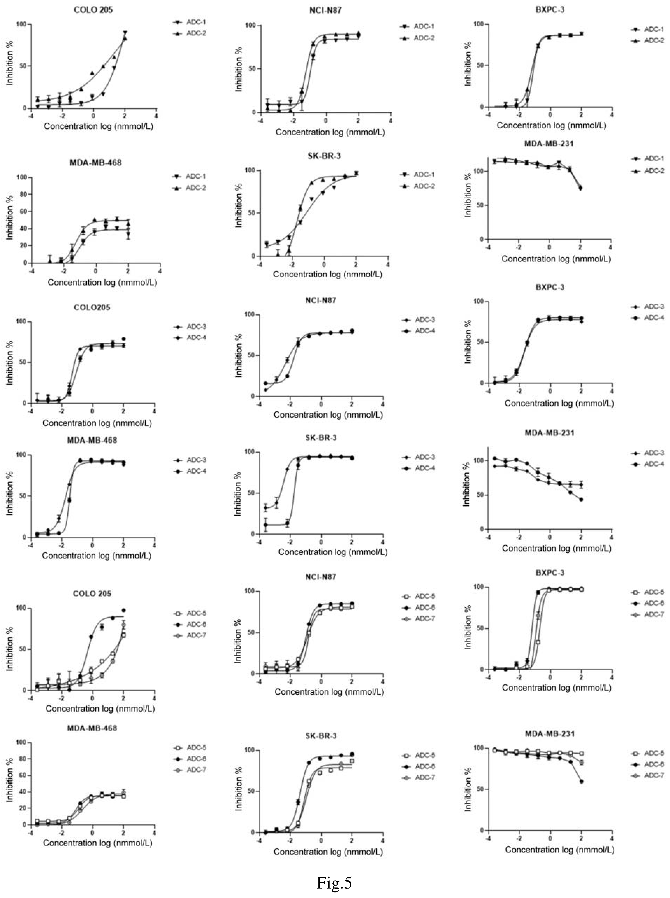

After 144 hours of drug treatment, the original culture medium was discarded, and a pre-mixed medium solution containing 10 µL MTT was added to each well. The cells were incubated for another 3-4 hours in a cell culture incubator, followed by the addition of 100 µL SDS lysis buffer to each well and incubation overnight at 37 °C to allow for complete cell lysis. Absorbance was measured at 570 nm. Data processing was performed using a GraphPad Prism 8, and the results are shown in Figure 5 and Table 2.

Results Analysis: Since the Trop2 target is widely distributed in normal tissues throughout the body, in terms of the selectivity of the actions of the ADC drugs, if the ADC drugs exhibits a high toxicity to Trop2-high expression cell lines with no or low toxicity to Trop2-low expression cell lines, they have improved safty. As shown in Figure 5 and Table 2, **ADC-1, ADC-2, ADC-5,** and **ADC-7** exhibited high cytotoxicity against TROP2-high expressing cells (BxPC-3, NCI-N87, MDA-MB-468, and SK-Br-3), but decreased cytotoxicity against TROP2-moderate or low-expressing cell lines (COLO205 and MDA-MB-231). In particular, **ADC-5** and **ADC-7** showed almost no cytotoxicity against TROP2-moderate or low-expressing cell lines, which may endow **ADC-5** with better safety performance. **ADC-3, ADC-4,** and **ADC-6** exhibited high cytotoxicity to cell lines with high (BxPC-3, NCI-N87, MDA-MB-468, and SK-Br-3) or moderate (COLO205) expression, while showing good safety to the moderate-low-expressing cell line MDA-MB-231, making them suitable for treating certain indications with moderate to high TROP2 expression.

**Table 2. IC₅₀ values of ADC-1~7**

| **Sample Name** | **BXPC-3** | **NCI-N87** | **COLO205** | **MDA-MB-468** | **SK-BR-3** | **MDA-MB-231** |
|---|---|---|---|---|---|---|
| **ADC-1** | 0.08 | 0.11 | 93.7 | 0.1 | 0.08 | >100 |
| **ADC-2** | 0.08 | 0.06 | 19.96 | 0.06 | 0.02 | >100 |
| **ADC-3** | 0.02 | 0.01 | 0.05 | 0.02 | 0.01 | -- |
| **ADC-4** | 0.02 | 0.02 | 0.09 | 0.03 | 0.02 | -- |
| **ADC-5** | 0.20 | 0.13 | >100 | 0.13 | 0.08 | >100 |
| **ADC-6** | 0.06 | 0.10 | 0.46 | 0.09 | 0.04 | >100 |
| **ADC-7** | 0.11 | 0.15 | >100 | 0.23 | 0.11 | >100 |

### Pharmacological Example 2: In vivo antitumor activity assay of ADC-5

BALB/c nude mouse xenograft models were established using gastric cancer cell line NCI-N87 and human breast squamous cell carcinoma cell line HCC1806. They were marked by ear punching and grouped according large, medium, small sizes, with 6 mice in each group.

**ADC-5** and **ADC-7** were selected for animal-level activity evaluation, with DS-1062 as a positive control and PBS as a negative control. All samples were sterilized for later use using a 0.22 mm filter membrane before administration.

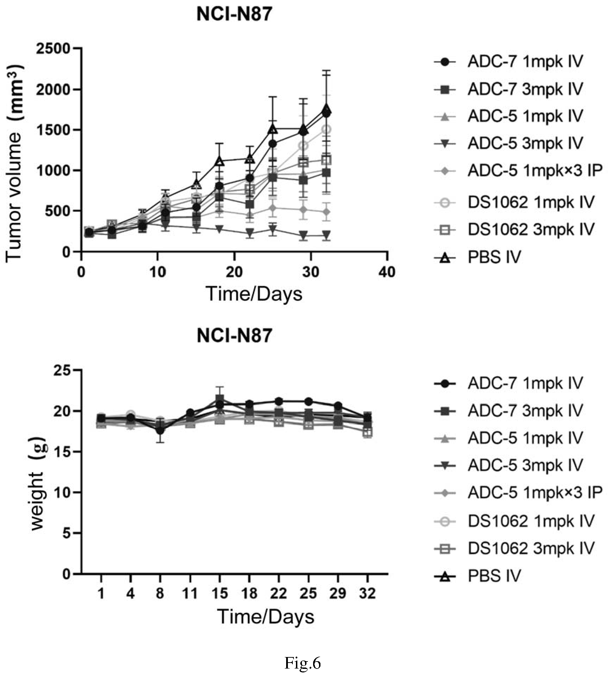

All samples were administered intravenously at doses of 1 mg/kg and 3 mg/kg respectively, with a single administration. Starting from the first administration, tumor size and mouse weight were measured twice a week using a caliper. The experimental procedure followed animal ethics requirements. The measured data were plotted and analyzed using GraphPad Prism 8 software. The results are shown in Figure 6: In the NCI-N87 tumor model, **ADC-5** at doses of 3 mg/kg and 1 mg/kg showed significantly better tumor-inhibiting activity than **ADC-7** and DS1062, demonstrating good in vivo tumor-inhibiting activity.

## Claims

1. An TROP2-targeting antibody-drug conjugate having a structure shown in the following Formula I: 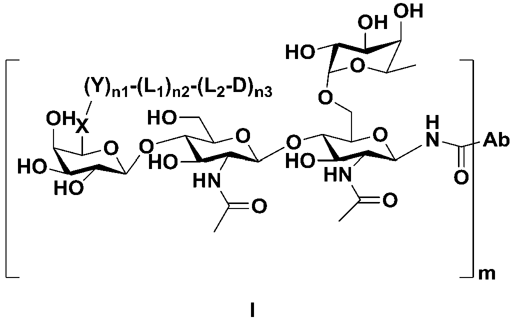
wherein m is 1 or 2, preferably 2;
Ab represents a TROP2-targeting antibody, which is an IgG having a conserved N-glycosylation site at N297 in the Fc region;
Xs are each independently -(CH=N-O)ₚ₀-(CH₂)ₚ₁; wherein p0 is 0 or 1, and p1 is an integer between 1 and 20, preferably an integer between 1 and 6;
Y represents -W-L₃-,
wherein W represents a structure formed by a reaction of orthorhombic groups to form a stable covalent bond, and the orthorhombic groups are selected from azide (N₃) group, dibenzocyclooctyl (DBCO), bicyclo[6.1.0]nonyne (BCN) group, and dibenzooctylene (DIBO) group;
L₃ represents a linking group selected from -(CH₂)ₚ₂-CO-, -( CH₂CH₂O)ₚ₃-CO-, and
-(CH₂)ₚ₄-CONH-(CH₂CH₂O)ₚ₅-(CH₂)ₚ₆-CO-; wherein p2, p3, p4, p5, and p6 are each independently selected from an integer between 1 and 20, preferably an integer between 2 and 6;
n1 is 0 or 1;
L₁ represents a linking group having 3 or more linking sites, such as a linking group derived from glutamic acid (Glu), β-glutamic acid, lysine (Lys), Lys-Glu dipeptide, or lysine-β-glutamic acid dipeptide, or a linking group derived from diethanolamine;
L₂ represents a short peptide linear linking group with 2 or more linking sites, with or without p-aminobenzylcarbamate, for example, it is a structural unit that is cleavable or non-cleavable in tumor microenvironment, such as Val-Cit-PAB, Val-Ala-PAB, Gly-Gly-Phe-Gly, or the like; further, optionally, L₁ and L₂ have a PEG chain attached to the end of their respective groups contained therein, for example, when L₁ is a linking group derived from Lys, a PEG chain is further attached to the amino group of lysine via an amide bond, and the PEG chain is, for example, -(CH₂CH₂O)ₚ₇-CH₃, wherein p7 is an integer between 2 and 20;
D represents a group derived from a toxin small molecule drug;
n2 is 0 and n3 is 1; or n2 is 1 and n3 is an integer between 2 and 4, for example, n2 is 1 and n3 is 2, 3, or 4, preferably n3 is 2;
Wherein n2 is 1, n3 of L₂-D groups are covalently linked to L₁, and n3 ≤ the number of linking sites of L₁ - 1.

2. The TROP2-targeting antibody-drug conjugate of claim 1, wherein the Ab is an antibody selected from IgG1, IgG2, or IgG4, preferably IgG1;
specifically, the antibody is a monoclonal antibody, a polyclonal antibody, a bifunctional antibody, a trifunctional antibody, a nanobody fused with an Fc domain, or a therapeutic or functional antibody from different species, wherein the species are selected from mouse, rat, sheep, rabbit, and human;
specifically, the antibody includes Sacituzumab and Datopotamab, preferably Sacituzumab.

3. The TROP2-targeting antibody-drug conjugate of claim 1, wherein W is selected from 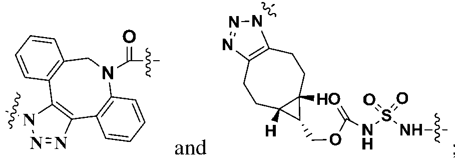 and/or
L₁ represents a linking group derived from glutamic acid (Glu), lysine (Lys), or diethanolamine;
preferably, L₁ is selected from 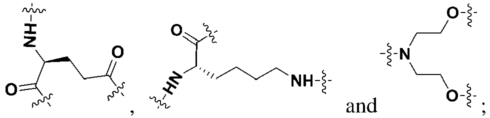 and/or
L₂ is 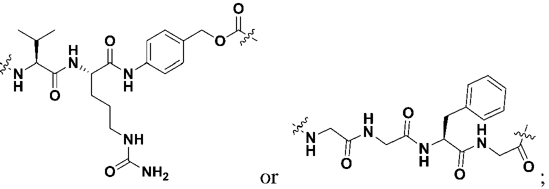 and/or
the toxin small molecule includes MMAE, MMAF, Eribulin, Dxd, Exatecan, DM1, or DM4; preferably, the toxin small molecule is a microtubule inhibitor MMAE or Eribulin; Specifically, D is selected from the following structures:

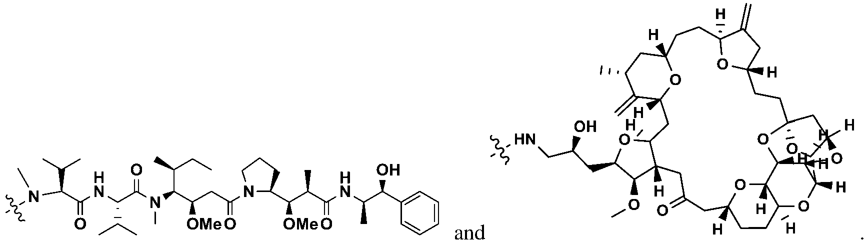

4. The TROP2-targeting antibody-drug conjugate of claim 1, wherein the TROP2-targeting antibody-drug conjugate has a structure shown in the following Formula **I-1 or** Formula **I-2:** 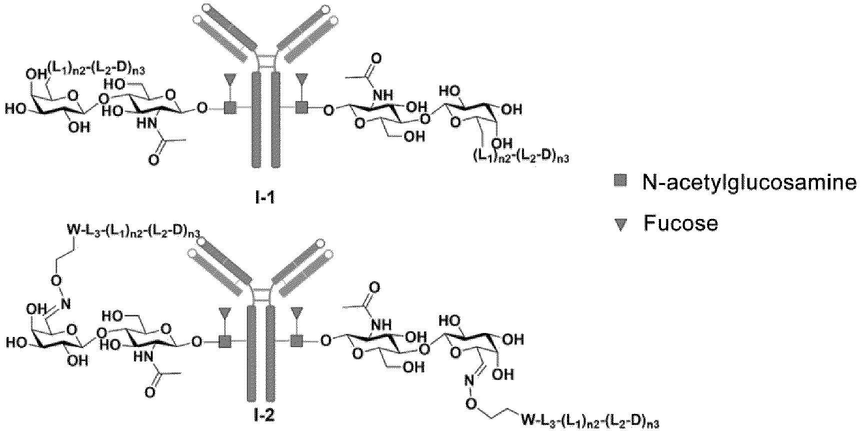 wherein W, L₁, L₂, L₃, D, n2, n3, and the antibody are defined as those in claim 1, respectively.

5. The TROP2-targeting antibody-drug conjugate of claim 1, wherein the TROP2-targeting antibody-drug conjugate has a DAR value of more than 0 and 4 or less, preferably 0.5 or more and 4 or less, more preferably, the DAR value is 1 to 4, and even more preferably, the DAR value is 2 or 4.

6. The TROP2-targeting antibody-drug conjugate of any one of claims 1 to 5, wherein the TROP2-targeting antibody-drug conjugate has the following structure: 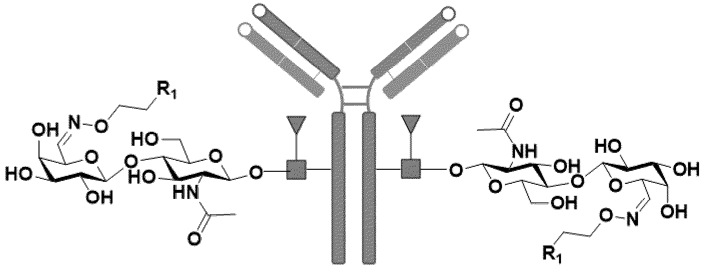
| ADC No. | R₁ |
|---|---|
| ADC-1 | 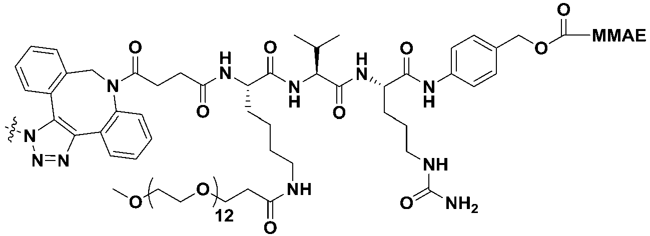 |
| ADC-2 | 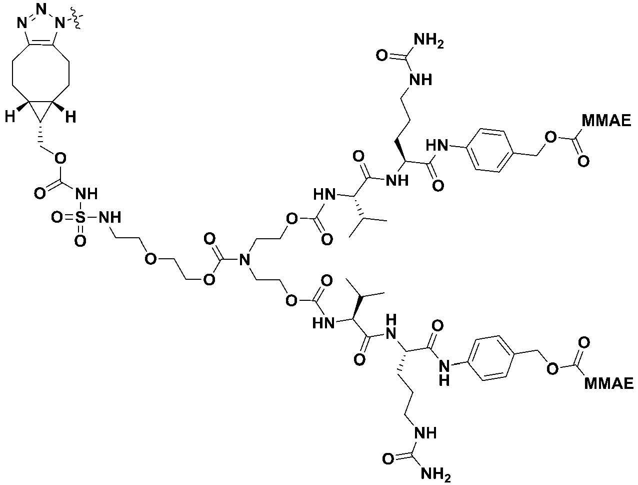 |
| ADC-3 | 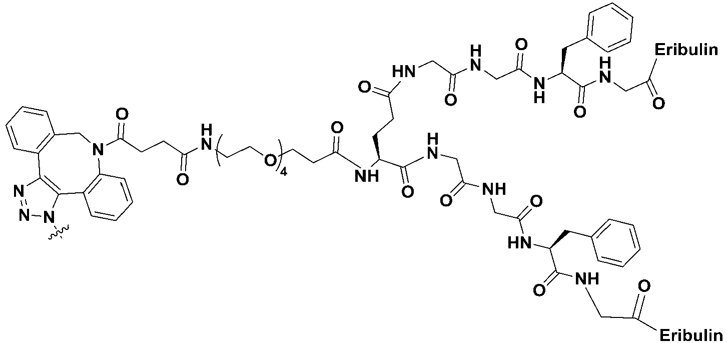 |
| ADC-4 | 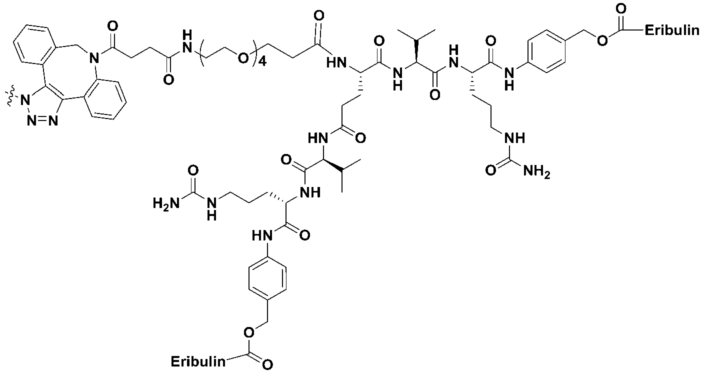 |
| ADC-6 | 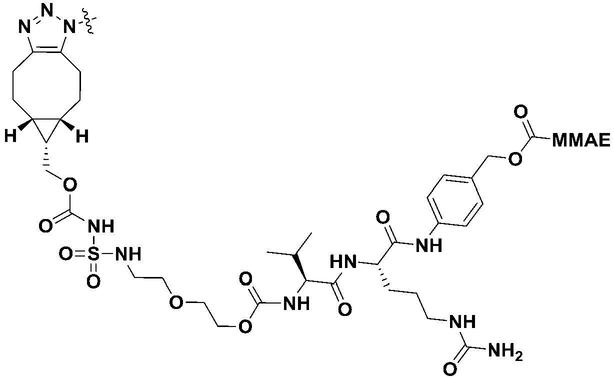 | 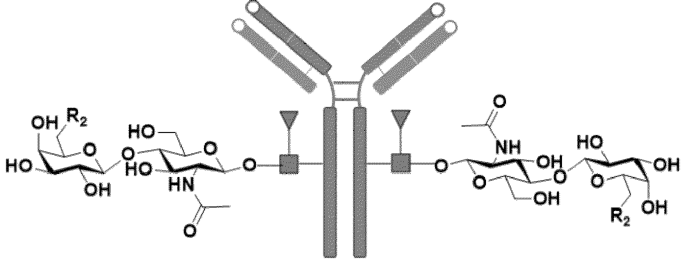
| ADC No. | R₂ |
|---|---|
| ADC-5 | 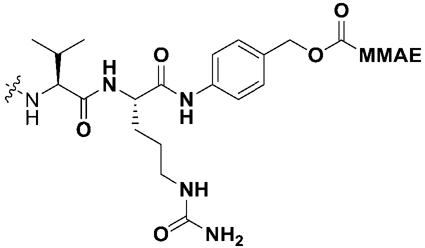 |
wherein in the above **ADC-1 to ADC-6,** the antibody is Sacituzumab; 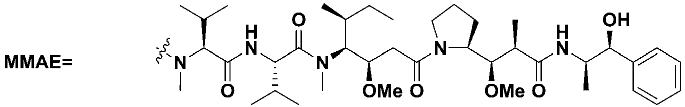 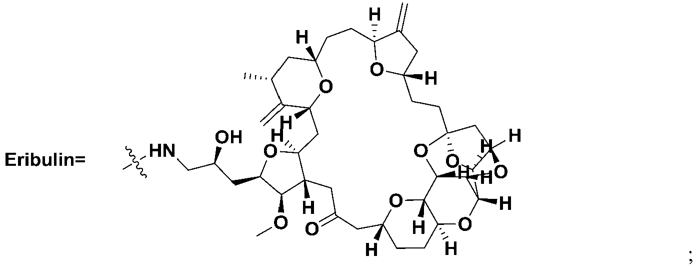 specifically, the TROP2-targeting antibody-drug conjugate has the structure of ADC-5.

7. A method for preparing the TROP2-targeting antibody-drug conjugate **I-1** or **I-2** of claim 4, the method is as follows: 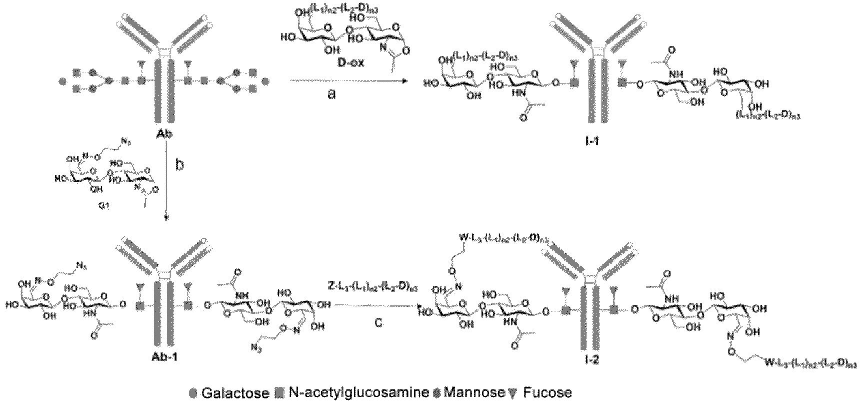
wherein Z represents an orthorhombic group capable of reacting with an azide group (N₃) to form the structure of W, such as a dibenzocyclooctyl (DBCO) group or a bicyclo[6.1.0]nonyne (BCN) group;
Ab, W, L₁, L₂, L₃, D, n2, and n3 are defined as described in claim 1, respectively;
Method 1: Preparation of antibody-drug conjugate **I-1**
Step a: reacting the toxin small molecule drug-disaccharide oxazoline complex D-ox with a wild-type antibody Ab in the presence of the wild-type endoglycosidase Endo-S2 to obtain the antibody-drug conjugate **I-1;**
Method 2: Preparation of Antibody-Drug Conjugate **I-2**
Step b: reacting the azide-disaccharide oxazoline **G1** with a wild-type antibody in the presence of the endoglycosidase Endo-S2 to obtain the non-natural glycoengineered antibody **Ab-1;**
Step c: reacting the non-natural glycoengineered antibody **Ab-1** with a toxin small molecule drug-linker Z-L₃-(L₁)ₙ₂-(L₂-D)ₙ₃ containing an orthorhombic group that can react with the azide group (N₃) to obtain the antibody-drug conjugate **I-2.**

8. The method of claim 7, wherein, in step a of method 1, the concentrations of the toxin small molecule drug-disaccharide oxazoline complex D-ox, the wild-type antibody, and the wild-type endoglycosidase Endo-S2 are 0.5 mM, 5 mg/mL, and 0.4 mg/mL, respectively; the reaction is carried out at 30 °C for 1 h, at pH 6.5; and/or
In step b of method 2, the concentrations of the azide-disaccharide oxazoline **G1,** the wild-type antibody, and the endoglycosidase Endo-S2 are 0.5 mM, 5 mg/mL, and 0.4 mg/mL, respectively;
the reaction is carried out at 30 °C for 1 h, at pH 6.5; and/or
In step c of method 2, the concentrations of the non-natural glycoengineered antibody **Ab-1** and
the toxin small molecule drug-linker are 5 mg/mL and 0.3 mM, respectively; the reaction is carried out at 37 °C in a 50 mM PB buffer system at pH 7.4.

9. The method of claim 7, wherein, in step a of method 1, the toxin small molecule drug-disaccharide oxazoline complex D-ox has the following structure: 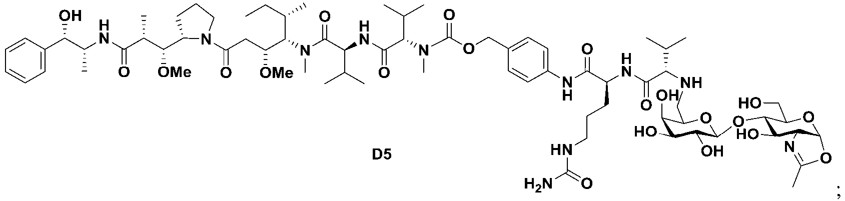 and/or
in step c of method 2, the toxin small molecule drug-linker Z-L₃-(L₁)ₙ₂-(L₂-D)ₙ₃ is selected from compounds with the following structures:

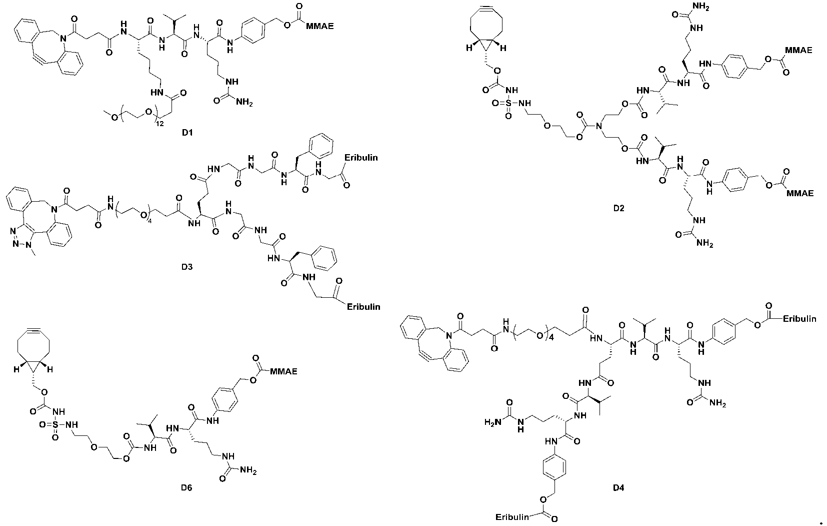

10. Use of the TROP2-targeting antibody-drug conjugates of any one of claims 1 to 6 and the antibody-drug conjugates prepared by the method of any one of claims 7 to 9 in the preparation of antitumor drugs;
preferably, the tumor is a TROP2-highly expressing tumor, preferably, the tumor includes: breast cancer (especially squamous cell carcinoma of the breast), gastric cancer, pancreatic cancer, colon cancer, bladder cancer, oral squamous cell carcinoma, ovarian cancer, urothelial carcinoma, lung cancer, especially breast cancer (especially squamous cell carcinoma of the breast) and gastric cancer.
